# EUROPÄISCHE PATENTSCHRIFT

(11) **EP 2 826 822 B1**
(45) Veröffentlichungstag und Bekanntmachung des Hinweises auf die Patenterteilung: **10.11.2021**
(21) Anmeldenummer: 14002208.8
(22) Anmeldetag: 27.06.2014
(51) Int. Cl.: C09C 1/00, A61Q 1/02, A61K 8/02, A61K 8/19, A61K 8/25, A61K 8/29, A61Q 1/10, A61Q 1/12, A61Q 19/00

(54) **Pigmentgemisch basierend auf sphärischen Partikeln**
Pigment mixture based on spherical particles
Mélange de pigment basé sur des particules sphériques

(30) Priorität: 19.07.2013 DE 102013012023
(43) Veröffentlichungstag der Anmeldung: 21.01.2015
(73) Patentinhaber: Merck Patent GmbH, 64293 Darmstadt (DE)
(72) Erfinder: Klein, Sylke, 64380 Rossdorf (DE); Heider, Lilia, 64579 Gernsheim (DE); Lorenz, Carsten, 64285 Darmstadt (DE); Schoen, Sabine, 45701 Herten (DE)

(56) Entgegenhaltungen:
- EP-A1- 1 063 265
- EP-A1- 2 316 891
- WO-A1-00/15720
- WO-A2-99/66883

## Beschreibung

Die vorliegende Erfindung betrifft ein Pigmentgemisch basierend auf sphärischen Partikeln mit einer definierten Teilchengrößenverteilung, sowie dessen Verwendung in Farben, Lacken, Druckfarben, Sicherheitsdruckfarben, Kunststoffen, keramischen Materialien, Gläsern, in kosmetischen Formulierungen, als Tracer, als Füllstoff und zur Herstellung von Pigmentpräparationen und Trockenpräparaten.

Als Pigmente werden Farbmittel bezeichnet, die im Anwendungsmedium unlöslich sind. Typische Beispiele für organische Pigmente sind Phthalocyanine, Diketo-Pyrrolopyrrole, Azopigmente und Chinacridone. Von den anorganischen Pigmenten seien die verschiedenen Arten von Eisenoxiden oder auch das Ultramarin genannt. Pigmente zeichnen sich gegenüber den im Anwendungsmedium löslichen Farbstoffen im Allgemeinen durch eine höhere chemische und photochemische Beständigkeit aus.

Als eine spezielle Form von Pigmenten können Füllstoffe betrachtet werden. Bei Füllstoffen steht nicht die Funktion "Farbgebung" im Vordergrund. Bei technischen Füllstoffen sind vielmehr Faktoren wie die Erhöhung der mechanischen Stabilität, die Abriebfestigkeit, die Wetterstabilität oder auch die Herstellkosten ausschlaggebend für ihre Verwendung.

Breite Anwendung finden Füllstoffe auch in kosmetischen Formulierungen. Beispielsweise können Puder bis zu 50 % Füllstoffe basierend auf der Endformulierung enthalten. In Lippenstiften sind 10-15 % an Füllstoffen und in Emulsionen 2-6 % an Füllstoffen typische Werte. Kosmetische Füllstoffe erfüllen ganz unterschiedliche Funktionen: in Foundations vermeiden sie durch den sogenannten Mattierungseffekt einen unerwünschten Fettglanz auf der Haut, in Pudern helfen sie z.B. das Schüttverhalten oder die Auftragseigenschaften auf der Haut zu verbessern. In Deo-Produkten wird das hohe Flüssigkeitsaufnahmevermögen von einigen Füllstoffen genutzt.

Füllstoffe bzw. Pigmente in der Kosmetik müssen vor ihrer Anwendung im zu pigmentierenden System in eine Form gebracht werden, die eine leichte Dispergierung und eine reproduzierbare Farbe ermöglicht. Diese Vorbehandlungen der Pigmente, beispielsweise Mahlen, die entscheidend die Qualität des Endproduktes beeinflussen, sind zeitaufwendig und teuer. Nachteilig ist weiterhin, dass beim Benetzen die Farbe des Pigments verändert wird. Für kosmetische Formulierungen müssen die Pigmente zusätzlich ein gutes Hautgefühl besitzen, welches die klassischen Füllstoffe nur in geringem Umfang zeigen.

Füllstoffe auf der Basis sphärischer Teilchen, insbesondere SiO₂-Kugeln, werden zunehmend in der Kosmetik eingesetzt, da sie einerseits der menschlichen Haut ein natürliches Aussehen verleihen und andererseits Falten weniger sichtbar machen können.

Anorganische sphärische Füllstoffe, die mit einer farbgebenden Schicht belegt sind, sind beispielsweise aus den Offenlegungsschriften JP 62-288662, JP 11-139926, JP 11-335240 und DE 199 29 109 bekannt. Aus der EP 2 316 891 A1 sind anorganische sphärische Pigmente bekannt, die auf der Oberfläche mit Agglomeraten beschichtet sind.

In der WO 00/15720 wird ein Pigmentgemisch basierend auf sphärischen SiO₂-Partikeln mit hoher Lichtdiffusion offenbart, wobei ein Teil der SiO₂-Kugeln mit TiO₂ und SiO₂ und der andere Teil mit TiO₂ und Fe₂O₃ beschichtet ist.

Aus der WO 99/66883 werden mit Metalloxiden, wie Titan-, Eisen- oder Zinkoxid, beschichtete SiO₂-Kugeln beschrieben, die eine finale SiO₂-Schicht aufweisen. Die so beschichteten SiO₂-Kugeln werden als Gemisch mit Interferenzpigmenten in kosmetischen Formulierungen eingesetzt.

Die aus dem Stand der Technik bekannten Füllstoffe basierend auf SiO₂-Kugeln zeigen ein relativ gutes Hautgefühl, besitzen aber den Nachteil, dass sie ein zu hohes Streuvermögen aufweisen. Der Grund dafür ist in der Struktur der Metalloxidschichten der kugelförmigen Füllstoffe zu suchen. Die funktionellen Pigmente aus dem Stand der Technik bestehen in der Regel aus sehr kleinen Partikeln, d. h., sie weisen Partikelgrößen von 0,5 - 100 nm auf, die in gleichmäßiger Anordnung die Oberfläche der Trägerkugeln belegen. An der Schichtoberfläche wird das Licht reflektiert, wodurch Glanz hervorgerufen wird. Gleichzeitig findet aber auch ein erhebliches Maß von Streuung statt, da sich auf der Schicht Einzelpartikel bilden, die als starke Streuzentren wirken. Als Folge dieser zwei gegenläufigen Effekte (Glanz und Streuung) wirken die Pigmente weiß und unnatürlich auf der Haut.

Aufgabe der vorliegenden Erfindung ist es daher ein funktionelles Pigment bereit zu stellen, das neben einem guten Hautgefühl, gleichzeitig eine gute Dispergierbarkeit in kosmetischen Formulierungen, chemische und photochemische Stabilität und eine reine, der Haut angepasste Farbe besitzt. Darüber hinaus soll das Pigment im Hautauftrag als reines Pulver, in Cremes, Emulsionen, Foundations, u.ä., ein weiches und ebenmäßiges und natürliches Erscheinungsbild der Haut zeigen. Es ist weiterhin erwünscht, dass das Pigment insbesondere flüssigen und pastösen Zubereitungen eine leichte Erhöhung der Festigkeit verleiht und die Stabilität der Zubereitung garantiert. Dadurch wird die Verteilbarkeit der kosmetischen Zubereitungen auf der Haut deutlich erleichtert. So können hochviskose Cremes, also feste Foundations, hergestellt werden, die dennoch eine sehr gute Verteilbarkeit auf der Haut bzw. ein sehr gutes Abtragsverhalten bei der Entnahme aus dem Container besitzen.

Neben diesen Produkteigenschaften ist eine einfache technische Herstellung des Pigments eine weitere Aufgabe der Erfindung. Auch die Einstellung bestimmter Eigenschaften, wie z.B. das Deckvermögen und die gezielt einstellbare Farbintensität, des Pigments sollen im Rahmen des Herstellungsverfahrens in einfacher Weise kontrolliert werden können.

Überraschenderweise wurde gefunden, dass Pigmentgemische basierend auf sphärischen Partikeln mit definierter Partikelgrößenverteilung, die auf der Oberfläche mit TiO₂ + SiO₂ oder TiO₂ + Eisenoxid beschichtet sind, eine bessere Einstellung des Deckvermögens und ein deutlich natürlicheres Erscheinungsbild der Haut erlauben und zudem ein besseres Hautgefühl als die Füllstoffe/Pigmente aus dem Stand der Technik aufweisen. Darüber hinaus zeigt das erfindungsgemäße Pigmentgemisch den gewünschten Einfluss auf die Textur und Stabilität, d. h. es wirkt leicht erhöhend auf die Viskosität von Emulsionen bzw. die Festigkeit von Foundations, ohne die Applikationseigenschaften ungünstig zu beeinflussen und es behält gleichzeitig die Stabilität der Zubereitung bei.

Gegenstand der Erfindung ist daher ein Pigmentgemisch basierend auf sphärischen Partikeln, dadurch gekennzeichnet, dass es mindestens zwei Komponenten A und B enthält, wobei
- Komponente A auf sphärischen Partikeln mit einer Teilchengrößenverteilung D₉₀ der unbeschichteten Partikel von 0,5 - 15 µm basiert, wobei die Partikel auf der Oberfläche zunächst mit TiO₂ und nachfolgend mit SiO₂ beschichtet sind,
   und
- Komponente B auf sphärischen Partikeln mit einer Teilchengrößenverteilung D₉₀ der unbeschichteten Partikel von 0,5 - 15 µm basiert, wobei die Partikel auf der Oberfläche zunächst mit TiO₂ und nachfolgend mit Eisenoxid oder mit einem Gemisch aus TiO₂ und Eisenoxid beschichtet sind.

Die erfindungsgemäßen sphärischen Pigmente zeigen gegenüber den Pigmenten aus dem Stand der Technik
- ein natürlicheres farblich angepasstes Erscheinungsbild der Haut
- eine leichtere Dispergierbarkeit
- eine verbesserte Verarbeitbarkeit
- ein in weiten Grenzen einstellbares Deckvermögen
- ein verbessertes Hautgefühl
- eine verbesserte Textur der kosmetischen Zubereitungen
- ein verbessertes Applikationsverhalten der kosmetischen Formulierungen.

Gegenstand der Erfindung ist weiterhin die Verwendung des erfindungsgemäßen Pigmentgemisches in Farben, Lacken, vorzugsweise in Industrielacken, Druckfarben, Sicherheitsdruckfarben, Kunststoffen, keramischen Materialien, Gläsern, als Tracer, als Füllstoff und insbesondere in kosmetischen Formulierungen. Weiterhin sind die erfindungsgemäßen Pigmente auch zur Herstellung von Pigmentpräparationen sowie zur Herstellung von Trockenpräparaten, wie z. B. Granulaten, Pearlets, Chips, Pellets, Würstchen, Briketts, etc., geeignet. Die Trockenpräparate finden insbesondere Anwendung in Druckfarben und in der Kosmetik.

Geeignete Basissubstrate sind sphärische Partikel, wie sie z. B. im Handel angeboten werden, u.a. von Sunjin Chemicals, Kobo, Ikeda, Asahi Glass, Miyoshi, Omega Materials, 3M, ABC NanoTech, China New Technology, PQ Corporation, Sibelco oder Evonik. Bevorzugte sphärische Partikel sind ausgewählt aus der Gruppe Magnesiumsilikat, Aluminiumsilikat, Alkalialuminiumsilikate, Erdalkalialuminiumsilikate und deren Kombinationen, SiO₂-Kugeln, Glaskugeln, Glashohlkugeln, Aluminiumoxid und polymere Kugeln aus Ethylen/ Acrylsäure-Copolymeren, Ethylen/ Methacrylat-Copolymeren, HDI/ Trimethylol Hexyllactone-Coplymeren, Nylon, Polyacrylate, Polymethylmethacrylat-Coplymere, Polyethylen, Polymethylsilsesquioxane und deren Kombinationen.

Besonders bevorzugte sphärische Substrate sind SiO₂-Kugeln, wie sie beispielsweise im Handel unter den Markennamen SUNSIL, MSS-500, SHERON, SUNSPHERE, Silicabeads SB, OMEGA-SIL, OMEGA-Spheres, SPHERICEL, Q-Cel, Ceramic Microspheres, Glasbubbles iM-K, SILNOS, SS-T4, SORBOSIL, AEROSIL, Flo-Beads, BPD, Daiamid, MSP, TOSPEARL, vertrieben werden.

Die per Stickstoffabsorption bestimmte BET-Oberfläche der geeigneten sphärischen Basispartikel beträgt in der Regel 1 - 1000, vorzugsweise 10 - 750, insbesondere 20 - 550 m²/g. Die BET-Oberfläche in dieser Patentanmeldung wird bestimmt nach DIN ISO 9277: 2003-05.

Die sphärischen Basissubstrate des erfindungsgemäßen Pigmentgemisches besitzen vorzugsweise einen Partikeldurchmesser im Bereich von 0,1 - 100 µm, insbesondere von 0,3 - 60 µm und ganz besonders bevorzugt von 0,5 - 15 µm. Die Partikel der Komponenten A und B können dabei gleich oder verschieden sein. Vorzugsweise sind die Partikel der Komponenten A und B sowohl in ihrer Zusammensetzung als auch in der Partikeldurchmesserverteilung und im D₉₀-Wert identisch und weisen lediglich eine unterschiedliche Beschichtung auf der jeweiligen Oberfläche auf.

Der D₉₀-Wert gibt den Partikeldurchmesser an, der mittels Laserbeugung ermittelt wird, welchen 90 Vol.% der Partikel der Gesamtheit aller Partikel maximal aufweisen.

Unter Beschichtung(en) sind in dieser Patentanmeldung die vollständige Belegung der sphärischen Basispartikel jeweils mit TiO₂ und SiO₂, im Fall der Komponente A und jeweils mit TiO₂ und Eisenoxid im Fall der Komponente B zu verstehen.

Die sphärischen Basispartikel der Komponente A sind auf der Oberfläche (1. Schicht) mit TiO₂ beschichtet. Das TiO₂ kann dabei in der Rutil- oder in der Anatas-Modifikation vorliegen, vorzugsweise liegt es als Anatas vor. Vorzugsweise weist die TiO₂-Schicht eine Schichtdicke von 10 - 500 nm, insbesondere von 25 - 400 nm und ganz besonders bevorzugt von 50 - 300 nm auf. Die Schichtdicke ist dabei nicht nur von den Belegungsparametern, sondern auch von der Kugelgröße der SiO₂-Kugeln abhängig. Je kleiner die Kugel und damit die Kugeloberfläche, um so dicker ist die aufgebrachte TiO₂-Schicht bei denselben vorliegenden Belegungsparametern. Eine Verdopplung des Kugeldurchmessers kann bereits zur Halbierung der TiO₂-Schichtdicke führen, was im oben genannten Bereich der Partikelgrößenverteilung von 0,5-15 µm gegeben ist.

Auf die TiO₂-Schicht wird nachfolgend eine SiO₂-Schicht appliziert. Diese finale SiO₂-Schicht dient u.a. zur Verbesserung der Dispergierbarkeit, der chemischen und photochemischen Stabilität und des Hautgefühls. Vorzugsweise weist die SiO₂-Schicht eine Schichtdicke von 10 - 500 nm, insbesondere von 25 - 400 nm und ganz besonders bevorzugt von 50 - 300 nm auf.

Die sphärischen Basispartikel der Komponente B sind auf der Oberfläche (1. Schicht) mit TiO₂ beschichtet. Das TiO₂ kann dabei in der Rutil- oder in der Anatas-Modifikation vorliegen, vorzugsweise liegt es als Anatas vor. Auf die TiO₂-Schicht wird nachfolgend eine Eisenoxidschicht, z.B. aus Fe₂O₃ oder Fe₃O₄, vorzugsweise eine Fe₂O₃-Schicht, appliziert. Die Schichtdicken der TiO₂- und der Eisenoxid-Schicht können dabei gleich oder verschieden sein.

Vorzugsweise weist die TiO₂-Schicht eine Schichtdicke von 50 - 400 nm, insbesondere von 200 - 400 nm und ganz besonders bevorzugt von 100 - 200 nm auf. Die Eisenoxid-Schicht hat vorzugsweise Schichtdicken von 5 - 300 nm, insbesondere von 10 - 200 nm und ganz besonders bevorzugt von 20 - 100 nm. Abhängig von der Schichtdicke und der anschließenden Calcinierung können verschiedene Farben von Gelb-, Braun- bis Rottönen eingestellt werden.

Die Beschichtung der Komponente B kann aber auch ein Gemisch aus TiO₂ und Eisenoxid sein. Vorzugsweise besteht dieses Gemisch aus TiO₂/Fe₂O₃ und/oder aus Pseudobrookit Fe₂TiO₅. Das TiO₂-/Eisenoxidgemisch weist vorzugsweise eine Schichtdicke von 55 - 700 nm auf und ganz besonders bevorzugt von 120 - 300 nm.

Besonders bevorzugte Komponenten A und B des erfindungsgemäßen Pigmentgemisches weisen folgende Belegung auf der Oberfläche auf:
sphärisches Basissubstrat + TiO₂ + SiO₂
sphärisches Basissubstrat + TiO₂ + Fe₂O₃
sphärisches Basissubstrat + TiO₂/Fe₂O₃
sphärisches Basissubstrat + Fe₂TiO₅.

Die Pigmente der Komponente A und die Pigmente der Komponente B können in jedem Verhältnis miteinander gemischt werden. Bevorzugt für die Farboptimierung in der jeweiligen Anwendung ist ein Mischungsverhältnis (Gewichtsteile) von Komponente A zu Komponente B von 99 : 1 bis 90 : 10, insbesondere von 98,5 : 1,5 bis 95 : 5 und ganz besonders bevorzugt von 98 : 2 bis 96 : 4. Das Mischungsverhältnis bezieht sich hierbei auf das Masse.

Das erfindungsgemäße Pigmentgemisch weist in der Regel eine Ölzahl von 10 - 200 g / 100g, insbesondere von 20 - 200 g / 100 g, ganz besonders bevorzugt von 50 - 150 g / 100 g, auf. Die Ölzahl in dieser Patentanmeldung wird bestimmt nach DIN ISO 787/5-1980 (E).

Die erfindungsgemäßen Pigmentgemische verbessern insbesondere die Textur von Kosmetika in der Weise, dass sie ein erleichtertes Auftragen und eine gleichmäßigere Verteilung auf der Haut erzielen und das Hautgefühl verbessern. Da das erfindungsgemäße Pigmentgemisch auf nichttoxischer mineralischer Basis aufgebaut ist und überwiegend anorganische Komponenten enthält, ist es sehr gut auf der Haut verträglich.

Die Belegung der sphärischen Basispartikel kann in einem Eintopfverfahren erfolgen. Die erfindungsgemäßen Pigmente lassen sich auf verschiedene Art und Weisen relativ einfach herstellen. Die sphärischen Partikel können durch nasschemische Beschichtung oder im CVD- oder PVD-Verfahren mit einer oder mehreren Beschichtungen belegt werden.

Vorzugsweise erfolgt die Belegung der sphärischen Basispartikel der Komponente A und/oder B im naßchemischen Verfahren durch hydrolytische Abscheidung der Metalloxide bzw. Metallhydroxide aus ihren Salzlösungen.

Die Metalloxidschichten auf den sphärischen Partikeln der Komponente A oder Komponente B werden vorzugsweise nasschemisch aufgebracht, wobei in der Regel die zur Herstellung von Perlglanzpigmenten entwickelten nasschemischen Beschichtungsverfahren angewendet werden können. Derartige Verfahren sind z. B. beschrieben in U.S. 3087828, U.S. 3087829, U.S. 3553001, DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017, DE 196 18 568, EP 0 659 843, oder auch in weiteren dem Fachmann bekannten Patentdokumenten und sonstigen Publikationen.

Bei der nasschemischen Beschichtung werden die sphärischen Partikel der Komponente A bzw. der Komponente B in Wasser suspendiert und mit einem oder mehreren hydrolysierbaren Metallsalzen in einem für die Hydrolyse geeigneten pH-Wert versetzt, der so gewählt wird, dass die Metalloxide bzw. Metalloxidhydrate direkt auf den Kugeln ausgefällt werden. Der pH-Wert wird üblicherweise durch gleichzeitiges Zudosieren einer Base oder Säure konstant gehalten. Anschließend werden die Pigmente abgetrennt, gewaschen und getrocknet, vorzugsweise bei 50-350 °C, insbesondere bei 80-150 °C, und gegebenenfalls calciniert, wobei die Glühtemperatur in Hinblick auf die jeweils vorliegende Beschichtung optimiert werden kann. In der Regel liegen die Glühtemperaturen zwischen 250 und 1100 °C, vorzugsweise zwischen 350 und 900 °C. Abhängig von der Glühtemperatur lassen sich Farbtöne in Gelb, Braun und Rot erzielen. Insbesondere bei Glühtemperaturen > 700 °C sind die Gelbtöne vorherrschend, da sich bei Komponente B Mischoxide aus Titanoxid und Eisenoxid, z.B. Pseudobrookit, bilden.

Falls gewünscht können die Pigmente nach Aufbringen einzelner Beschichtungen abgetrennt, gewaschen und ggf. geglüht werden, um dann zur Auffällung der weiteren Schichten oder zur Einstellung des finalen Farbtons, z.B. Zugabe der TiO₂/Fe₂O₃-beschichteten sphärischen Partikel zu der TiO₂/SiO₂-beschichteten Partikelsuspension, wieder resuspendiert zu werden.

Weiterhin kann die Beschichtung auch in einem Wirbelbettreaktor durch Gasphasenbeschichtung erfolgen, wobei z. B. die in EP 0 045 851 und EP 0 106 235 zur Herstellung von Perlglanzpigmenten vorgeschlagenen Verfahren entsprechend angewendet werden können.

Für das Aufbringen einer finalen SiO₂-Schicht wird bevorzugt das in der DE 196 18 569 beschriebene Verfahren verwendet. Zur Herstellung der SiO₂-Schicht wird vorzugsweise Natrium- oder Kaliumwasserglaslösung eingesetzt.

Wenn die gewünschte Menge des Schichtmaterials aufgefällt ist, wird die Reaktion unterbrochen und ein für die Aufarbeitung geeigneter pH-Wert, z.B. pH 5, eingestellt.

Zur Aufarbeitung werden die beschichteten sphärischen Partikel abfiltriert, mit Wasser gewaschen und vorzugsweise bei Temperaturen von 50 - 150 °C für eine Dauer von 1 - 20 h getrocknet und gegebenenfalls bei Temperaturen von 350 - 950 °C für 0,1 - 2 h kalziniert. Abschließend wird das Pigment der Komponente A bzw. der Komponente B oder das Pigmentgemisch gesiebt. Im letzten Schritt werden die getrennt aufgearbeiteten Komponenten A und B im gewünschten Verhältnis miteinander gemischt, wenn die Mischung nicht schon - wie oben beschrieben - in Suspension erfolgte.

Der Farbton und das Deckvermögen der Komponenten A und B kann in weiten Grenzen durch unterschiedliche Wahl der Beschichtungsstoffe und der Belegungsmengen bzw. der daraus resultierenden Schichtdicken sowie den angewendeten Glühtemperaturen variiert werden. Die Feinabstimmung für einen bestimmten Farbton kann über die reine Mengenwahl hinaus durch visuell oder messtechnisch kontrolliertes Anfahren der gewünschten Farbe erreicht werden.

Zur Erhöhung der Licht-, Wasser- und Wetterstabilität empfiehlt es sich häufig, in Abhängigkeit vom Einsatzgebiet, das Pigment der Komponente A bzw. der Komponente B einer Nachbeschichtung oder Nachbehandlung zu unterziehen. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage. Durch diese Nachbeschichtung wird die chemische und photochemische Stabilität weiter erhöht oder die Handhabung des Pigmentgemisches, insbesondere die Einarbeitung in unterschiedliche Medien erleichtert. Zur Verbesserung der Benetzbarkeit, Dispergierbarkeit und/oder Verträglichkeit mit den Anwendermedien können funktionelle Beschichtungen aus Al₂O₃ oder ZrO₂ oder deren Gemische auf die Pigmentoberfläche aufgebracht werden. Weiterhin sind organische Nachbeschichtungen möglich, z.B. mit Silanen, wie beispielsweise beschrieben in der EP 0090259, EP 0 634 459, WO 99/57204, WO 96/32446, WO 99/57204, U.S. 5,759,255, U.S. 5,571,851, WO 01/92425 oder in J.J. Ponjee, Philips Technical Review, Vol. 44, No. 3, 81 ff. und P.H. Harding J.C. Berg, J. Adhesion Sci. Technol. Vol. 11 No. 4, S. 471-493.

Gegenstand der Erfindung ist auch ein Verfahren zur Herstellung des erfindungsgemäßen Pigmentgemisches.

Das erfindungsgemäße Pigmentgemisch ist mit einer Vielzahl von Farbsystemen kompatibel, vorzugsweise aus dem Bereich der Lacke, Farben und Druckfarben. Für die Herstellung der Druckfarben für z. B. den Tiefdruck, Flexodruck, Offsetdruck, Offsetüberdrucklackierung, ist eine Vielzahl von Bindern, insbesondere wasserlösliche Typen, geeignet, wie sie z. B. von den Firmen BASF, Marabu, Pröll, Sericol, Hartmann, Gebr. Schmidt, Sicpa, Aarberg, Siegwerk, GSB-Wahl, Follmann, Ruco oder Coates Screen INKS GmbH vertrieben werden. Die Druckfarben können auf Wasserbasis oder Lösemittelbasis aufgebaut sein.

Mit dem erfindungsgemäßen Pigmentgemisch im angegebenen Partikelgrößenbereich von 0,5-15 µm lassen sich besonders wirksame Effekte wie Glättung von Oberflächen und Ausgleichung von Unebenheiten (Falten, Poren, Vertiefungen, Mikrorisse, etc., in den verschiedenen Anwendungsmedien erzielen, z. B. in kosmetischen Formulierungen, wie z. B. Nagellacken, Lippenstiften, Presspudern, Gelen, Lotionen, Seifen, Zahnpasta, in Lacken, in Industrielacken und Pulverlacken, sowie in Kunststoffen und in der Keramik.

Aufgrund des guten Hautgefühls und der sehr guten Hautadhäsion ist das erfindungsgemäße Pigmentgemisch insbesondere als Füllstoff in der dekorativen Kosmetik, aber auch für Personal Care Applications, wie z.B. Body Lotions, Emulsionen, Seifen, Shampoos, etc., geeignet.

Das erfindungsgemäße Pigmentgemisch weist eine stabilisierende Wirkung auf, wie sie z.B. in Cremes, Emulsionen und Lotions gewünscht wird.

Es versteht sich von selbst, dass für die verschiedenen Anwendungszwecke das erfindungsgemäße Pigmentgemisch auch vorteilhaft in Abmischung mit z. B.
- Metalleffektpigmenten, z. B. auf der Basis von Eisen- oder Aluminiumplättchen;
- Perlglanzpigmenten auf der Basis von metalloxidbeschichteten synthetischen Glimmer-Plättchen, natürlichen Glimmerplättchen, Glas-Plättchen, Al₂O₃-Plättchen, Fe₂O₃-Plättchen oder SiO₂-Plättchen;
- Interferenzpigmenten auf der Basis von metalloxidbeschichteten synthetischen Glimmer-Plättchen, natürlichen Glimmerplättchen, Glas-Plättchen, Al₂O₃-Plättchen, Fe₂O₃-Plättchen oder SiO₂-Plättchen;
- goniochromatischen Pigmenten;
- Mehrschichtpigmenten (enthaltend vorzugsweise 2, 3, 4, 5 oder 7 Schichten) auf der Basis von metalloxidbeschichteten synthetischen Glimmer-Plättchen, natürlichen Glimmerplättchen, Glas-Plättchen, Al₂O₃-Plättchen, Fe₂O₃-Plättchen oder SiO₂-Plättchen;
- organischen Farbstoffen;
- organischen Pigmenten;
- anorganischen Pigmenten, wie z.B. transparenten und deckenden Weiß-, Bunt- und Schwarzpigmenten;
- plättchenförmigen Eisenoxiden;
- Ruß
eingesetzt werden kann.

Das erfindungsgemäße Pigmentgemisch kann in jedem Verhältnis mit handelsüblichen Pigmenten und/oder weiteren handelsüblichen Füllstoffen gemischt werden.

Als handelsübliche Füllstoffe sind z.B. zu nennen natürlicher und synthetischer Glimmer, Nylon Powder, reine oder gefüllte Melaminharze, Talkum, Gläser, Kaolin, Oxide oder Hydroxide von Aluminium, Magnesium, Calcium, Zink, BiOCI, Bariumsulfat, Calciumsulfat, Calciumcarbonat, Magnesiumcarbonat, Kohlenstoff, Bornitrid sowie physikalische oder chemische Kombinationen dieser Stoffe. Bezüglich der Partikelform des Füllstoffes gibt es keine Einschränkungen. Sie kann den Anforderungen gemäß z.B. plättchenförmig, sphärisch oder nadelförmig sein.

Selbstverständlich kann das erfindungsgemäße Pigmentgemisch in den Formulierungen auch mit jeder Art von kosmetischen Roh-und Hilfsstoffen kombiniert werden. Dazu gehören u.a. Öle, Fette, Wachse, Filmbildner, Konservierungsmittel und allgemein anwendungstechnische Eigenschaften bestimmende Hilfsstoffe, wie z.B. Verdicker und rheologische Zusatzstoffe, wie z.B. Bentonite, Hektorite, Siliziumdioxide, Ca-Silicate, Gelatinen, hochmolekulare Kohlenhydrate und/oder oberflächenaktive Hilfsmittel, etc.

Die das erfindungsgemäße Pigmentgemisch enthaltende Formulierung kann dem lipophilen, hydrophilen oder hydrophoben Typ angehören. Bei heterogenen Formulierungen mit diskreten wässrigen und nichtwässrigen Phasen kann das erfindungsgemäße Pigmentgemisch in jeweils nur einer der beiden Phasen enthalten oder auch über beide Phasen verteilt sein.

Die pH-Werte der Formulierungen können zwischen 1 und 14, bevorzugt zwischen 2 und 11 und besonders bevorzugt zwischen 4 und 10 liegen.

Den Konzentrationen des erfindungsgemäßen Pigmentgemisches in der Formulierung sind keine Grenzen gesetzt. Sie können ― je nach Anwendungsfall - zwischen 0,001 (rinse-off-Produkte, z.B. Duschgele) - und 60 % liegen. Das erfindungsgemäße Pigmentgemisch kann weiterhin auch mit kosmetischen Wirkstoffen kombiniert werden. Geeignete Wirkstoffe sind z.B. Insect Repellents, UV A/BC-Schutzfilter (z.B. OMC, B3, MBC), Anti-Ageing-Wirkstoffe, Vitamine und deren Derivate (z.B. Vitamin A, C, E, etc.), Selbstbräuner (z.B. DHA, Erytrolose, u.a.) sowie weitere kosmetische Wirkstoffe, wie z.B. Bisabolol, LPO, Ectoin, Emblica, Allantoin, Bioflavanoide und deren Derivate.

Gegenstand der vorliegenden Erfindung sind ebenfalls Formulierungen, insbesondere kosmetische Formulierungen, die neben dem erfindungsgemäßen Pigmentgemisch mindestens einen Bestandteil ausgewählt aus der Gruppe der Absorptionsmittel, Adstringenzien, antimikrobiellen Stoffe, Antioxidantien, Antiperspirantien, Antischaummitteln, Antischuppenwirkstoffe, Antistatika, Bindemittel, biologischen Zusatzstoffen, Bleichmitteln, Chelatbildnern, Desodorierungsmitteln, Emollentien, Emulgatoren, Emulsionsstabilisatoren, Farbstoffen, Feuchthaltemitteln, Filmbildnern, Füllstoffen, Geruchsstoffen, Geschmacksstoffen, Insect Repellents, Konservierungsmitteln, Korrosionsschutzmitteln, kosmetischen Ölen, Lösungsmitteln, Oxidationsmitteln, pflanzlichen Bestandteilen, Puffersubstanzen, Reduktionsmitteln, Tensiden, Treibgasen, Trübungsmitteln, UV-Filtern und UV-Absorbern, Vergällungsmitteln, Viskositätsreglern, Parfüm und Vitaminen enthalten.

Gegenstand der Erfindung ist auch die Verwendung des erfindungsgemäßen Pigmentgemisches in Farben, Lacken, Druckfarben, Sicherheitsdruckfarben, Kunststoffen, keramischen Materialien, Gläsern, als Tracer, in kosmetischen Formulierungen und zur Herstellung von Pigmentpräparationen und Trockenpräparaten.

Die nachfolgenden Beispiele sollen die Erfindung näher erläutern ohne sie jedoch zu beschränken.

### Beispiele

### Beispiel 1

### Herstellung der Komponente A

200 g der sphärischen SiO₂₋Partikel mit einem D₉₀-Wert von 4-8 µm werden bei einem pH-Wert von 2-2,5 mit 300-330 g TiCl₄-Lösung (400 g TiCl₄/l) und anschließend bei einem pH-Wert von 7,8-8,2 mit 300-310 g Natronwasserglas (13-15 Gew.% SiO₂) beschichtet und nachgerührt. Die aufgefällten TiO₂₋ und SiO₂-Mengen betragen jeweils 12-16 Gew.% bezogen auf die Gesamtmasse der beschichteten weißen Partikel.

### Herstellung der Komponente B

Parallel dazu werden 100 g der sphärischen SiO₂-Partikel mit einem D₉₀-Wert von 4-8 µm in einem anderen Reaktionsgefäß bei einem pH-Wert von 2-2,5 mit 200 g TiCl₄-Lösung (400 g TiCl₄/l) und anschließend bei einem pH-Wert von 3-3,5 mit 100-130 g FeCl₃-Lösung (218 g FeCl₃) beschichtet und nachgerührt. Die Einstellung der gewünschten Farbe (von ockergelb bis rötlich) erfolgt durch in-situ Messungen der Lab-Werte. Die aufgefällten TiO₂₋ bzw. Fe₂O₃-Mengen betragen 16-19 Gew.% bzw. 7-10 Gew.% bezogen auf die Gesamtmasse der beschichteten gelb/rötlichen Partikel.

Nach der Belegung wird die Suspension salzfrei gewaschen, abfiltriert, und die Partikel werden bei 110°C getrocknet und gesiebt.

Von diesen gelb/rötlichen sphärischen Partikeln (Komponente B) werden - je nach gewünschter Farbe des Endprodukts - 2-10 Gew.% bezogen auf die Masse der Komponente A zur Suspension der Komponente A hinzugegeben und mind. 15 min gerührt. Nach dem Absitzen der Komponenten A und B wird die überstehende Lösung abdekantiert, die sphärischen Teilchen abfiltriert und salzfrei gewaschen. Nach der Trocknung bei 110 °C werden 100 g des Endprodukts bei 600 °C für 45 min kalziniert und gesiebt.

Je nach Gehalt der Komponente B können Farbtöne von aprikot (2-4 Gew.%) über blass rosa (5-6 Gew.%) bis kräftig rosa (7-10 Gew.%) eingestellt werden.

Die Einstellung des Mischverhältnisses der Komponente A und der Komponente B kann auch durch Einwaage der trockenen Pigmente erfolgen.

### Beispiel 2

Die wie in Beispiel 1 beschriebenen Mischungen aus Komponente A und Komponente B können durch die Wahl der Glühtemperatur farblich eingestellt werden. Beginnend bei Temperaturen von 600 bis 750 °C verschiebt sich der Farbton von rosa/bräunlich/aprikot immer mehr ins Gelbliche bis ab 700 °C das Endprodukt gelblich-weiß erscheint. Hier setzt die Pseudobrookit-Bildung (Fe₂TiO₅) ein.

### Anwendungsbeispiele

### Beispiel A1: Eye shadow Gel

| **Rohstoff** | | **INCI** | **[%]** |
|---|---|---|---|
| Phase A | | | |
| Timiron Super Gold | (1) | MICA, Cl 77891 (TITANIUM DIOXIDE) | 15,00 |
| Pigmentgemisch aus Beispiel 1 | | | 7,00 |
| Carbopol Ultrez 21 | (2) | Acrylates/C10-30 Alkyl Acrylate crosspolymer | 0,30 |
| Aloe Vera Powder regular 200x | (3) | Aloe Barbadensis | 0,05 |
| Citronensäure Monohydrat | (1) | Citric Acid | 0,00 |
| Wasser, demineralisiert | | Aqua (Water) | 55,87 |
| Phase B | | | |
| Triethanolamin 90 % Care | (4) | Triethanolamine, Aqua (Water) | 0,78 |
| Germaben II | (5) | Propylene Glycol, Diazolidinyl Urea, Methylparaben, Propylparaben | 1,00 |
| Glycerin, wasserfrei | (1) | Glycerin | 2,00 |
| Wasser, demineralisiert | | Aqua (Water) | 13,00 |
| Phase C | | | |
| Lubrajel DV | (6) | Propylene Glycol, Polyglycerylmethacrylate | 5,00 |

### Herstellung:

Aloe Vera Puder im Wasser der Phase A lösen, dann alle Pigmente und das Pigmentgemisch und die restlichen Inhaltsstoffe bis auf das Carbopol zufügen und dispergieren. Mit einigen Tropfen Citronensäure ansäuern, um die Viskosität zu vermindern, dann Carbopol unter Rühren einstreuen. Nach vollständiger Lösung die vorgelöste Phase B langsam einrühren (nicht homogenisieren) und anschließend Phase C zugeben. Falls erforderlich, den pH-Wert zwischen 7,0-7,5 mit Citronensäurelösung einstellen.

Man erhält eine wasserbasierende Eye Shadow Gel Formulierung mit Aloe Vera. (Schnellsttrocknend und gut mit den Fingern aufzutragen.)

### Bezugsquellen:

(1) Merck KGaA/Rona^{®}
(2) Noveon
(3) Terry Laboratoires, Inc.
(4) BASF AG
(5) ISP Global Technologies
(6) Guardian

### Beispiel A2: Creamy Eyeshadow

| **Rohstoff** | | **INCI** | **[%]** |
|---|---|---|---|
| Phase A | | | |
| Colorona Light Blue | (1) | MICA, Cl 77891 (TITANIUM DIOXIDE) | 10,00 |
| | | Cl 77510 (FERRIC FERROCYANIDE) | |
| Pigmentgemisch aus Beispiel 1 | | | 15,00 |
| Talkum | (1) | Talc | 12,00 |
| Phase B | | | |
| Crodamol PMP | (2) | PPG-2Myristyl Ether Propionate | 32,80 |
| Miglyol 812 N | (3) | Caprylic/Capric Triglyceride | 12,00 |
| Syncrowax HGLC | (2) | C18-36 Acid Triglyceride | 10,00 |
| Syncrowax HRC | (2) | Tribehenin | 3,00 |
| Parteck^{®} LUB STA | (1) | Stearic Acid | 3,00 |
| Antaron V-216 | (4) | PVP/Hexadecene Copolymer | 2,00 |
| Oxynex^{®} K flüssig | (1) | PEG-8, Tocopherol, Ascorbyl Palmitate, Ascorbic Acid, Citric Acid | 0,10 |
| Propyl-4-hydroxybenzoat | (1) | Propylparaben | 0,10 |

### Herstellung:

Phase B auf ca. 80 °C erhitzen bis alles geschmolzen ist und unter Rühren auf 65 °C abkühlen. Unter Rühren nun die Inhaltsstoffe der Phase A zugeben und die Masse bei 65 °C in das vorgesehene Packmittel gießen. Auf Raumtemperatur abkühlen lassen.

### Bezugsquellen:

(1) Merck KGaA/Rona^{®}
(2) Croda GmbH
(3) Sasol Germany GmbH
(4) ISP Global Technologies

### Beispiel A3: Gesichtspuder

| **Rohstoff** | | **INCI** | **[%]** |
|---|---|---|---|
| Phase A | | | |
| Pigmentgemisch aus Beispiel 1 | | | 20,00 |
| Unipure Yellow LC 182 | (1) | Cl 77492 (Iron Oxides) | 1,20 |
| Unipure Red LC 381 | (1) | Cl 77491 (Iron Oxides) | 0,20 |
| Unipure Brown LC 889 | (1) | Cl 77491 (Iron Oxides) Cl 77499 (Iron Oxides) | 0,30 |
| Magnesiumstearat | (2) | Magnesium Stearate | 2,00 |
| Talkum | (2) | Talc | 71,90 |
| Phase B | | | |
| RonaCare^{®} all-rac-alpha- | (2) | Tocopherylacetate | 0,30 |
| Tocopherylacetat Parfümöl 200 529 | (3) | Parfum | 0,30 |
| Eutanol G | (4) | Octyldodecanol | 3,70 |
| Propyl-4-hydroxybenzoat | (2) | Propylparaben | 0,10 |

### Herstellung:

Die Bestandteile der Phase A in den Mixer (z. B. La Moulinette von Moulinex) geben und 2 × 10 Sekunden mixen. Die Mischung in ein Becherglas überführen, Phase B hinzugeben und mit dem Spatel vorab verrühren. Die Mischung aus Phase A und Phase B wiederum in den Mixer geben und 3 × 10 Sekunden zu einer homogenen Phase verarbeiten.

Der Pressdruck für eine Puderpfanne mit 36 mm Durchmesser beträgt ca. 25 bar.

### Bezugsqellen:

(1) Les Colorants Wackherr
(2) Merck KGaA/Rona^{®}
(3) Fragrance Resources
(4) Cognis GmbH

### Beispiel A4: Mattifying Foundation

| **Rohstoff** | | **INCI** | **[%]** |
|---|---|---|---|
| Phase A | | | |
| Wasser, demineralisiert | | Aqua (Water) | 57,89 |
| Pigmentgemisch aus Beispiel 1 | | | 6,00 |
| Glycerin (87 % reinst) | (1) | Glycerin, Aqua (Water) | 5,00 |
| RonaCare^{®} Ectoin | (1) | Ectoin | 0,30 |
| Keltrol CG-SFT | (2) | Xanthan Gum | 0,15 |
| Triethanolamin 90% Care | (3) | Triethanolamine, Aqua (Water) | 0,13 |
| Phase B | | | |
| Kronos 1001 | (4) | Cl 77891 (Titanium Dioxide) | 4,92 |
| Unipure Yellow LC 182 | (5) | Cl 77492 (Iron Oxides) | 1,60 |
| Unipure Red LC 381 | (5) | Cl 77491 (Iron Oxides) | 0,20 |
| Unipure Brown LC 889 | (5) | Cl 77491 (Iron Oxides) Cl 77499 (Iron Oxides) | 0,20 |
| Unipure Blue LC 686 | (5) | Cl 77007 (Ultramarin Blue) | 0,08 |
| Phase C | | | |
| Miglyol 812N | (6) | Caprylic/Capric Triglyceride | 7,00 |
| Eutanol G | (7) | Octyldodecanol | 4,00 |
| Montanov 202 | (8) | Arachidyl Alcohol, Behenyl Alcohol, Arachidylglucoside | 4,00 |
| Avocadoöl | (9) | Persea Gratissima (Avocado Oil) | 2,00 |
| Eusolex^{®} 9020 | (1) | Butyl Methoxydibenzoylmethane | 1,50 |
| Hydrolite-5 | (10) | Pentylene Glycol | 1,20 |
| Bentone Gle GTCC V | (11) | Stearalkonium Hectorite, Propylene Carbonate, Caprylic/Capric Triglyceride | 1,00 |
| RonaCare^{®} all-rac-alpha-Tocopherylacetat | (2) | Tocopherylacetate | 0,50 |
| Phenonip | (12) | Phenoxyethanol, Butylparaben, Ethylparaben, Propylparaben, Methylparaben | 0,40 |
| Oxynex^{®} K flüssig | (1) | PEG-8, Tocopherol, Ascorbyl Palmitate, Ascorbic Acid, Citric Acid | 0,03 |
| Phase D | | | |
| Simulgel EG | (8) | Sodium Acrylate/Sodium Acryloyldimethyltaurate Copolymer, Isohexadecane, Polysorbate 80 | 0,60 |
| Phase E | | | |
| Wasser, demineralisiert | | Aqua (Water) | 1,00 |

### Herstellung:

Keltrol langsam ins Wasser der Phase A geben und dispergieren. Restliche Bestandteile der Phase A unter Rühren einstreuen. Die Bestandteile der Phase B zur Phase A geben und mit dem Ultra-Turrax T25 (Stufe rot-blau, 13500-20500 Upm) 3 min homogenisieren und auf Agglomerate prüfen. Phase A/B und Phase C separat auf 75 °C erhitzen. Unter Rühren Phase C zu Phase A/B geben und 2 min mit dem Ultra-Turrax T25 (Stufe gelb-grün, 8000-9500 Upm) homogenisieren. Zwischen 55-60 °C Phase D zugeben, bei 40 °C Phase E zugeben, unter weiterem Rühren auf Raumtemperatur abkühlen; pH-Wert auf 7,0 mit 30 %iger Citronensäure einstellen. Dann in geeignete Behältnisse abfüllen. Man erhält eine leichte, schwach deckende Foundation, die für alle Hauttypen geeignet ist. Avocadoöl, Vitamin-E-Acetat und das zellschützende RonaCare^{®} Ectoin unterstützen die hautpflegende Wirkung.

### Bezugsquellen:

(1) Merck KGaA/Rona^{®}
(2) C.P. Kelco
(3) BASF AG
(4) Kronos International Inc.
(5) Les Colorants Wackherr
(6) Sasol Germany GmbH
(7) Cognis GmbH
(8) Seppic
(9) Gustav Heess GmbH
(10) Symrise
(11) Elementis Specialities
(12) Clariant GmbH

### Beispiel A5: Body Lotion

| **Rohstoff** | | **INCI** | **[%]** |
|---|---|---|---|
| Phase A | | | |
| Aloe Vera Gel 10x declorized | (1) | ALOE BARBADENSIS | 2,00 |
| D-Panthenol Pigmentgemisch aus Beispiel 1 | (2) | PANTHENOL | 0,40 6,00 |
| RonaCare^{®} Allantoin | (3) | ALLANTOIN | 0,20 |
| Glycerin, wasserfrei | (3) | GLYCERIN | 4,00 |
| Wasser, demineralisiert | | AQUA (WATER) | 67,57 |
| Phase B | | | |
| Protelan AGL 95/C | (4) | SODIUM COCOYL GLUTAMATE | 6,00 |
| Cosmacol EMI | (5) | DI-C12-13 ALKYL MALATE | 3,00 |
| Eutanol G | (6) | Octyldodecanol | 3,00 |
| Jojobaöl | (7) | SIMMONDSIA CHINENSIS (JOJOBA OIL) | 1,50 |
| Tegosoft TN | (8) | C12-15 Alkyl Alkyl benzoate | 1,50 |
| Carbopol ETD 2020 | (9) | Acrylates/C10-30 Alkyl Acrylate Crosspolymer | 0,60 |
| Phenonip | (10) | Phenoxyethanol, Butylparaben, Ethylparaben, Propylparaben, Methylparaben | 0,60 |
| RonaCare^{®} Bisabolol | (3) | Bisabolol | 0,50 |
| RonaCare^{®} all-rac-alpha-Tocopherylacetat | (3) | Tocopherylacetate | 0,50 |
| Oxynex^{®} ST Liquid | (3) | Diethylhexyl Syringylidenemalonate, Caprylic/Capric Triglyceride | 0,50 |
| Cremophor RH 410 | (11) | PEG-40 Hydrogenated Castor Oil | 0,30 |
| Oxynex^{®} K flüssig | | PEG-8, Tocopherol, Ascorbyl Palmitate, Ascorbic Acid, Citric Acid | 0,03 |
| Phase C | | | |
| Parfümöl Lifetime DH 10255/1 | (12) | Parfum | 0,50 |
| Phase D | | | |
| Wasser, demineralisiert | | Aqua (Water) | 1,00 |
| Germal 115 | (13) | Imidazolidinyl Urea | 0,30 |

### Herstellung:

Aloe Vera und RonaCare^{®} Allantoin im Wasser der Phase A unter Rühren vorlösen, dann die anderen Bestandteile der Phase A zugeben und auf 60 °C erhitzen, Jojobaöl, Oxynex K flüssig, Cosmacol EMI, Eutanol G und Tegosoft TN in einem Rührgefäß vorlegen, dann Carbopol mit der Dispergierscheibe (ca. 700 U/min, 20 min) dazu homogen einarbeiten. Dann die restlichen Bestandteile der Phase B zufügen und alles zu einer homogenen Mischung verrühren, wobei Protelan AGL 95/C erst ganz zum Schluss zur Phase B zugegeben wird, um einen übermäßigen Lufteintrag zu vermeiden. Phase A bei 60 °C mit Hilfe der Dispergierscheibe langsam in Phase B (RT) einemulgieren. Phasen C und D zufügen, danach 4 min mit dem Ultra-Turrax T50, Stufe 4 homogenisieren. Auf Raumtemperatur abkühlen.
pH (23 °C) = 5,5 ― 6,0
Viskosität: Brookfield DV II + Helipath, Spindel C, 5 Rpm, 24 °C=11200 mPa·s

### Bezugsquellen:

(1) Terry Laboratoires
(2) Alfa Aesar GmbH & Co. KG
(3) Merck KGaA/Rona^{®}
(4) Zschimmer & Schwarz GmbH & Co.
(5) Nordmann, Rassmann GmbH & Co.
(6) Cognis GmbH
(7) Gustav Heess GmbH
(8) Evonik Goldschmidt GmbH
(9) Noveon
(10) Clariant GmbH
(11) BASF AG
(12) Parfex
(13) ISP Global Technologies

## Patentansprüche

1. Pigmentgemisch basierend auf sphärischen Partikeln, **dadurch gekennzeichnet, dass** es mindestens zwei Komponenten A und B enthält, wobei
- Komponente A auf sphärischen Partikeln mit einer Teilchengrößenverteilung D₉₀ der unbeschichteten Partikel von 0,5 - 15 µm basiert, wobei die Partikel auf der Oberfläche zunächst mit TiO₂ und nachfolgend mit SiO₂ beschichtet sind,
und
- Komponente B auf sphärischen Partikeln mit einer Teilchengrößenverteilung D₉₀ der unbeschichteten Partikel von 0,5 - 15 µm basiert, wobei die Partikel auf der Oberfläche zunächst mit TiO₂ und nachfolgend mit Eisenoxid oder mit einem Gemisch aus TiO₂ und Eisenoxid beschichtet sind.

2. Pigmentgemisch nach Anspruch 1, **dadurch gekennzeichnet, dass** die sphärischen Partikel ausgewählt sind aus der Gruppe Magnesiumsilikat, Aluminiumsilikat, Alkalialuminiumsilikate, Erdalkalialuminiumsilikate und deren Kombinationen, SiO₂-Kugeln, Glaskugeln, Glashohlkugeln, Nylon, Aluminiumoxid und polymere Kugeln aus Ethylen/Acrylsäure-Copolymeren, Ethylen/Methacrylat-Copolymeren, HDI/Trimethylol Hexyllactone-Copolymeren, Nylon, Polyacrylate, Polymethylmethacrylat-Copolymere, Polyethylen, Polymethylsilsesquioxane und deren Kombinationen.

3. Pigmentgemisch nach Anspruch 1 oder 2, **dadurch gekennzeichnet, dass** die sphärischen Basispartikel der Komponente A und/oder B aus SiO₂ bestehen.

4. Pigmentgemisch nach mindestens einem der Ansprüche 1 bis 3, **dadurch gekennzeichnet, dass** die sphärischen Partikel der Komponente A und der Komponente B jeweils aus SiO₂ bestehen.

5. Pigmentgemisch nach mindestens einem der Ansprüche 1 bis 4, **dadurch gekennzeichnet, dass** das Eisenoxid der Komponente B Fe₂O₃ ist.

6. Pigmentgemisch nach mindestens einem der Ansprüche 1 bis 5, **dadurch gekennzeichnet, dass** die Komponente A auf der Oberfläche mit TiO₂ in der Anatas-Modifikation beschichtet ist.

7. Pigmentgemisch nach mindestens einem der Ansprüche 1 bis 6, **dadurch gekennzeichnet, dass** die TiO₂-Schicht der Komponente A eine Schichtdicke von 10 - 500 nm aufweist.

8. Pigmentgemisch nach mindestens einem der Ansprüche 1 bis 7, **dadurch gekennzeichnet, dass** die SiO₂-Schicht der Komponente A eine Schichtdicke von 10 - 500 nm aufweist.

9. Pigmentgemisch nach mindestens einem der Ansprüche 1 bis 8, **dadurch gekennzeichnet, dass** die Komponente B auf der Oberfläche mit TiO₂ in der Anatas-Modifikation beschichtet ist.

10. Pigmentgemisch nach mindestens einem der Ansprüche 1 bis 9, **dadurch gekennzeichnet, dass** die TiO₂-Schicht der Komponente B eine Schichtdicke von 50 - 400 nm aufweist.

11. Pigmentgemisch nach mindestens einem der Ansprüche 1 bis 10, **dadurch gekennzeichnet, dass** die Eisenoxid-Schicht der Komponente B eine Schichtdicke von 5 - 300 nm aufweist.

12. Pigmentgemisch nach mindestens einem der Ansprüche 1 bis 11, **dadurch gekennzeichnet, dass** das Gemisch aus TiO₂ und Eisenoxid der Komponente B eine Schichtdicke von 55 - 700 nm aufweist.

13. Pigmentgemisch nach mindestens einem der Ansprüche 1 bis 12, **dadurch gekennzeichnet, dass** die Komponenten A und B im Massenverhältnis (Gewichtsteile) 99 : 1 bis 90 : 10 gemischt werden.

14. Pigmentgemisch nach einem oder mehreren der Ansprüche 1 bis 13, **dadurch gekennzeichnet, dass** die Komponente A und/oder die Komponente B zur Erhöhung der Licht-, Temperatur- und Wetterstabilität zusätzlich eine äußere Schutzschicht aufweist.

15. Verfahren zur Herstellung des Pigmentgemisches nach einem oder mehreren der Ansprüche 1 bis 14, **dadurch gekennzeichnet, dass** die sphärischen Partikel der Komponenten A und B jeweils durch nasschemische Beschichtung oder im CVD- oder PVD-Verfahren mit einer oder mehreren Beschichtungen belegt werden und nachfolgend die Komponente A und die Komponente B miteinander gemischt werden.

16. Verwendung des Pigmentgemisches nach einem oder mehreren der Ansprüche 1 bis 14 in Farben, Lacken, Druckfarben, Sicherheitsdruckfarben, Kunststoffen, keramischen Materialien, Gläsern, in kosmetischen Formulierungen, als Tracer, als Füllstoff und zur Herstellung von Pigmentpräparationen und Trockenpräparaten.

17. Formulierungen enthaltend ein Pigmentgemisch nach einem oder mehreren der Ansprüche 1 bis 14.

18. Formulierungen nach Anspruch 17, **dadurch gekennzeichnet, dass** sie neben dem Pigmentgemisch nach einem oder mehreren der Ansprüche 1 bis 14 mindestens einen Bestandteil ausgewählt aus der Gruppe der Absorptionsmittel, Adstringenzien, antimikrobiellen Stoffe, Antioxidantien; Antiperspirantien, Antischaummitteln, Antischuppenwirkstoffe, Antistatika, Bindemittel, biologischen Zusatzstoffen, Bleichmitteln, Chelatbildnern, Desodorierungsmitteln, Emollentien, Emulgatoren, Emulsionsstabilisatoren, Farbstoffen, Feuchthaltemitteln, Filmbildnern, Füllstoffen, Geruchsstoffen, Geschmacksstoffen, Insect Repellents, Konservierungsmitteln, Korrosionsschutzmitteln, kosmetischen Ölen, Lösungsmitteln, Oxidationsmitteln, pflanzlichen Bestandteilen, Puffersubstanzen, Reduktionsmitteln, Tensiden, Treibgasen, Trübungsmitteln, UV-Filtern und UV-Absorbern, Vergällungsmitteln, Viskositätsreglern, Parfüm und Vitaminen enthalten.

## Claims

1. Pigment mixture based on spherical particles, **characterised in that** it comprises at least two components A and B, where
- component A is based on spherical particles having a particle-size distribution D₉₀ of the uncoated particles of 0.5 - 15 µm, where the particles are coated on the surface firstly with TiO₂ and subsequently with SiO₂,
and
- component B is based on spherical particles having a particle-size distribution D₉₀ of the uncoated particles of 0.5 - 15 µm, where the particles are coated on the surface firstly with TiO₂ and subsequently with iron oxide or with a mixture of TiO₂ and iron oxide.

2. Pigment mixture according to Claim 1, **characterised in that** the spherical particles are selected from the group magnesium silicate, aluminium silicate, alkali-metal aluminium silicates, alkaline-earth metal aluminium silicates and combinations thereof, SiO₂ spheres, glass spheres, hollow glass spheres, nylon, aluminium oxide and polymeric spheres comprising ethylene-acrylic acid copolymers, ethylene-methacrylate copolymers, HDI-trimethylol hexyl lactone copolymers, nylon, polyacrylates, polymethyl methacrylate copolymers, polyethylene, polymethylsilsesquioxanes and combinations thereof.

3. Pigment mixture according to Claim 1 or 2, **characterised in that** the spherical base particles of component A and/or B consist of SiO₂.

4. Pigment mixture according to at least one of Claims 1 to 3, **characterised in that** the spherical particles of component A and component B each consist of SiO₂.

5. Pigment mixture according to at least one of Claims 1 to 4, **characterised in that** the iron oxide of component B is Fe₂O₃.

6. Pigment mixture according to at least one of Claims 1 to 5, **characterised in that** component A is coated on the surface with TiO₂ in the anatase modification.

7. Pigment mixture according to at least one of Claims 1 to 6, **characterised in that** the TiO₂ layer of component A has a layer thickness of 10 - 500 nm.

8. Pigment mixture according to at least one of Claims 1 to 7, **characterised in that** the SiO₂ layer of component A has a layer thickness of 10 - 500 nm.

9. Pigment mixture according to at least one of Claims 1 to 8, **characterised in that** component B is coated on the surface with TiO₂ in the anatase modification.

10. Pigment mixture according to at least one of Claims 1 to 9, **characterised in that** the TiO₂ layer of component B has a layer thickness of 50 - 400 nm.

11. Pigment mixture according to at least one of Claims 1 to 10, **characterised in that** the iron oxide layer of component B has a layer thickness of 5 - 300 nm.

12. Pigment mixture according to at least one of Claims 1 to 11, **characterised in that** the mixture of TiO₂ and iron oxide of component B has a layer thickness of 55 - 700 nm.

13. Pigment mixture according to at least one of Claims 1 to 12, **characterised in that** components A and B are mixed in the weight ratio (parts by weight) 99 : 1 to 90 : 10.

14. Pigment mixture according to one or more of Claims 1 to 13, **characterised in that** component A and/or component B additionally has an outer protective layer in order to increase the light, temperature and weather stability.

15. Process for the preparation of the pigment mixture according to one or more of Claims 1 to 14, **characterised in that** the spherical particles of components A and B are in each case covered with one or more coatings by wet-chemical coating or by the CVD or PVD process, and component A and component B are subsequently mixed with one another.

16. Use of the pigment mixture according to one or more of Claims 1 to 14 in paints, coatings, printing inks, security printing inks, plastics, ceramic materials, glasses, in cosmetic formulations, as tracer, as filler and for the preparation of pigment preparations and dry preparations.

17. Formulations comprising a pigment mixture according to one or more of Claims 1 to 14.

18. Formulations according to Claim 17, **characterised in that**, besides the pigment mixture according to one or more of Claims 1 to 14, they comprise at least one constituent selected from the group of the absorbents, astringents, antimicrobial substances, antioxidants, antiperspirants, antifoaming agents, antidandruff active compounds, antistatics, binders, biological additives, bleaches, chelating agents, deodorisers, emollients, emulsifiers, emulsion stabilisers, dyes, humectants, film formers, fillers, fragrances, flavours, insect repellents, preservatives, anticorrosion agents, cosmetic oils, solvents, oxidants, plant constituents, buffer substances, reducing agents, surfactants, propellant gases, opacifiers, UV filters and UV absorbers, denaturing agents, viscosity regulators, perfume and vitamins.

## Revendications

1. Mélange de pigments basé sur des particules sphériques, **caractérisé en ce qu'**il comprend au moins deux composants A et B, où
- le composant A est basé sur des particules sphériques ayant une distribution granulométrique D₉₀ des particules non revêtues de 0,5 - 15 µm, où les particules sont revêtues en surface d'abord par du TiO₂ puis par du SiO₂,
et
- le composant B est basé sur des particules sphériques ayant une distribution granulométrique D₉₀ des particules non revêtues de 0,5 - 15 µm, où les particules sont revêtues en surface d'abord par du TiO₂ puis par de l'oxyde de fer ou par un mélange de TiO₂ et d'oxyde de fer.

2. Mélange de pigments selon la revendication 1, **caractérisé en ce que** les particules sphériques sont choisies dans le groupe constitué par le silicate de magnésium, le silicate d'aluminium, les silicates d'aluminium et de métal alcalin, les silicates d'aluminium et de métal alcalino-terreux et des combinaisons de ceux-ci, les sphères de SiO₂, les sphères en verre, les sphères en verre creuses, le nylon, l'oxyde d'aluminium et les sphères polymères comprenant des copolymères d'éthylène-acide acrylique, des copolymères d'éthylène-méthacrylate, des copolymères de HDI-triméthylol hexyl lactone, du nylon, des polyacrylates, des copolymères de polyméthacrylate de méthyle, du polyéthylène, des polyméthylsilsesquioxanes et des combinaisons de ceux-ci.

3. Mélange de pigments selon la revendication 1 ou 2, **caractérisé en ce que** les particules de base sphériques de composant A et/ou B sont constituées de SiO₂.

4. Mélange de pigments selon au moins l'une des revendications 1 à 3, **caractérisé en ce que** les particules sphériques de composant A et de composant B sont chacune constituées de SiO₂.

5. Mélange de pigments selon au moins l'une des revendications 1 à 4, **caractérisé en ce que** l'oxyde de fer du composant B est Fe₂O₃.

6. Mélange de pigments selon au moins l'une des revendications 1 à 5, **caractérisé en ce que** le composant A est revêtu en surface par du TiO₂ selon la modification anatase.

7. Mélange de pigments selon au moins l'une des revendications 1 à 6, **caractérisé en ce que** la couche de TiO₂ du composant A présente une épaisseur de couche de 10 - 500 nm.

8. Mélange de pigments selon au moins l'une des revendications 1 à 7, **caractérisé en ce que** la couche de SiO₂ du composant A présente une épaisseur de couche de 10 - 500 nm.

9. Mélange de pigments selon au moins l'une des revendications 1 à 8, **caractérisé en ce que** le composant B est revêtu en surface par du TiO₂ selon la modification anatase.

10. Mélange de pigments selon au moins l'une des revendications 1 à 9, **caractérisé en ce que** la couche de TiO₂ du composant B présente une épaisseur de couche de 50 - 400 nm.

11. Mélange de pigments selon au moins l'une des revendications 1 à 10, **caractérisé en ce que** la couche d'oxyde de fer layer du composant B présente une épaisseur de couche de 5 - 300 nm.

12. Mélange de pigments selon au moins l'une des revendications 1 à 11, **caractérisé en ce que** le mélange de TiO₂ et d'oxyde de fer du composant B présente une épaisseur de couche de 55 - 700 nm.

13. Mélange de pigments selon au moins l'une des revendications 1 à 12, **caractérisé en ce que** les composants A et B sont mélangés selon un rapport pondéral (parties en poids) allant de 99 : 1 à 90 : 10.

14. Mélange de pigments selon l'une ou plusieurs parmi les revendications 1 à 13, **caractérisé en ce que** le composant A et/ou le composant B possède en outre une couche protectrice externe afin d'augmenter la stabilité à la lumière, à la température et aux intempéries.

15. Procédé de préparation du mélange de pigments selon l'une ou plusieurs parmi les revendications 1 à 14, **caractérisé en ce que** les particules sphériques des composants A et B sont dans chaque cas revêtues par un ou plusieurs revêtements par revêtement chimique humide ou par le procédé CVD ou PVD, et le composant A et le composant B sont ensuite mélangés l'un avec l'autre.

16. Utilisation du mélange de pigments selon l'une ou plusieurs parmi les revendications 1 à 14 dans les peintures, les revêtements, les encres d'impression, les encres d'impression de sécurité, les matières plastiques, les matériaux céramiques, les verres, dans les formulations cosmétiques, comme traceur, comme charge et pour la préparation de préparations de pigments et de préparations sèches.

17. Formulations comprenant un mélange de pigments selon l'une ou plusieurs parmi les revendications 1 à 14.

18. Formulations selon la revendication 17, **caractérisées en ce que**, outre le mélange de pigments selon l'une ou plusieurs parmi les revendications 1 à 14, elles comprennent au moins un constituant choisi dans le groupe constitué par les absorbants, les astringents, les substances antimicrobiennes, les antioxydants, les antitranspirants, les agents antimousse, les composés actifs antipelliculaires, les agents antistatiques, les liants, les additifs biologiques, les agents javellisants, les agents chélatants, les désodorisants, les émollients, les émulsifiants, les stabilisateurs d'émulsion, les colorants, les humectants, les agents filmogènes, les charges, les fragrances, les arômes, les insectifuges, les conservateurs, les agents anticorrosion, les huiles cosmétiques, les solvants, les oxydants, les constituants végétaux, les substances tampon, les agents réducteurs, les agents tensioactifs, les gaz propulseurs, les opacifiants, les filtres anti-UV et les absorbants d'UV, les agents dénaturants, les régulateurs de viscosité, les parfums et les vitamines.
